# NEUE EUROPÄISCHE PATENTSCHRIFT

(11) **EP 1 762 230 B2**
(45) Veröffentlichungstag und Bekanntmachung des Hinweises auf die Entscheidung über den Einspruch: **20.04.2016**
(45) Hinweis auf die Patenterteilung: 14.10.2009
(21) Anmeldenummer: 06016747.5
(22) Anmeldetag: 10.08.2006
(51) Int. Cl.: A61K 9/20, A61K 31/529, A61P 35/02

(54) **Filmtablette oder Granulat enthaltend ein Pyridylpyrimidin**
Filmtablet or granulate comprising a pyridylpyrimidine
Tablette enrobé ou granulate comprenante un pyridylpyrimidine

(30) Priorität: 15.08.2005 CH 13332005
(43) Veröffentlichungstag der Anmeldung: 14.03.2007
(73) Patentinhaber: Siegfried International AG, 4800 Zofingen (CH)
(72) Erfinder: Röhrich, Lambert Tillmann, 4310 Rheinfelden (CH); Müller, Beat W., Therwil (CH)
(74) Vertreter: Braun, André jr.

(56) Entgegenhaltungen:
- WO-A-03/090720
- US-B1- 6 894 051
- H. A. LIEBERMAN ET AL.: 'Pharmaceutical Dosage Forms: Tablets', Bd. 1, 1980, MARCEL DEKKER INC., NEW YORK - BASEL Seiten 112,150 - 173
- USP 24, 2000, Seiten 2117 and 2118
- W. LUND: 'The Pharmaceutical Codex', Bd. 12, 1994, THE PARMACEUTICAL PRESS, LONDON Seiten 2 - 11
- P. KLEINBUDDE: 'Roll compaction/dry granulation: pharmaceutical applications' EUROPEAN JOUNAL OF PHARMACEUTICALS AND BIOPHARMACEUTICS Bd. 58, 2004, Seiten 317 - 326

## Beschreibung

Die vorliegende Erfindung betrifft eine stabile, oral applizierbare Verabreichungsform, vorzugsweise in Form von Filmtabletten und Granulaten, für die orale Verabreichung, enthaltend mindestens eine Pyridylpyrimidinverbindung oder ein pharmazeutisch annehmbares Salz einer solchen Verbindung, insbesondere 4-(4-methyl-piperazin-1-ylmethyl)-N-[4-methyl-3-(4-(3-pyridin-3-yl-pyrimdin-2-ylamino)-phenyl]-benzamid (Imatinib) oder ein pharmazeutisch annehmbares Salz davon. Ein bevorzugtes Salz von Imatinib ist das Salz der Methansulfonäure, nachstehend Imatinib-Monomethansulfonat oder Imatinib-Mesylat genannt, entsprechend der folgenden Verbindung der Formel (I):

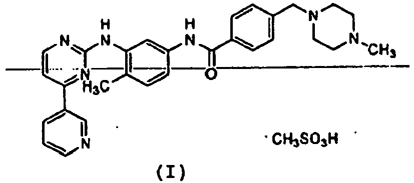

Die Wirksubstanz 4-(4-methyl-piperazin-1-ylmethyl)-N-[4-methyl-3-(4-(3-pyridin-3-yl-pyrimdin-2-ylamino)-phenyl]-benzamid und deren pharmazeutisch annehmbarer Salze in wasserfreier oder hydratisierter Form, deren Herstellung sowie deren pharmazeutische Wirkungen sind bekannt. So ist es aus EP 0 998 473 bekannt, Imatinib Mesylat in der Kristallform alfa (α) und der Kristallform beta (β) herzustellen, wobei die alfa-Form als hygroskopisch bezeichnet wird. Ferner wird festgehalten, dass die alfa-Form als nadelförmige Kristalle vorliegt und deshalb für die Tablettierung infolge ungünstiger Fliesseigenschaften nicht besonders gut geeignet ist.

Es hat sich gezeigt, dass ausgewählte medizinische Behandlungen, wie beispielsweise die Verabreichung als Tyrosin-Kinase-Hemmer zur Therapie von Leukämie, die Verabreichung von vergleichsweise hohen täglichen Dosen im Bereich von 100 mg bis 800 mg, insbesondere 400 mg bis 800 mg, Wirkstoff (Imatinib) erfordern. Dies bedeutet, dass Tabletten hergestellt werden müssen, welche einen vergleichsweise hohen Wirkstoffgehalt, vorzugsweise im Bereich von 25 Gew.-% bis 80 Gew.-% aufweisen, damit die Tablette nicht zu gross wird.

In WO 03/090720 werden Tabletten mit hohem Wirkstoffgehalt an Imatinib-Mesylat für die orale Verabreichung beschrieben, welche jedoch alle mittels konventioneller Feuchtgranulierung hergestellt werden. Nur mittels Feuchtgranulierung kann gemäss diesem Dokumentoffensichtlich die notwendige Härte und Abriebfestigkeit der Tablette einerseits und die genügende Bioverfügbarkeit des Wirkstoffs andererseits erreicht werden, wobei Imatinib-Mesylat vorzugsweise in der beta-Kristallform verwendet wird.

Es wurde nun gefunden, dass es möglich ist, feste Verabreichungsformen, insbesondere Filmtabletten und Granulate, enthaltend eine Pyridylpyrimidinverbindung oder ein pharmazeutisch annehmbares Salz dieser Verbindung, vorzugsweise Imatinib oder ein pharmazeutisch annehmbares Salz von Imatinib, vorzugsweise Imatinib-Mesylat, sowohl in der alfa-Form als auch in der beta-Form, vorzugsweise in der alfa-Form, mittels Verpressung der Ausgangsstoffe, d.h. den Wirkstoff zusammen mit den Zusatzstoffen, herzustellen, wenn man, vorgängig zur Verpressung der Ausgangsstoffe, mindestens einen der Ausgangsstoffe trockengranuliert, vorzugsweise verdichtet bzw. kompaktiert, vorzugsweise mit Walzen kompaktiert bzw. verdichtet, derart dass, gegebenenfalls nach anschliessendem Mischen sämtlicher Ausgangsstoffe, eine fliessfähige Mischung aller Ausgangsstoffe erhalten wird. Dabei kann man, vorgängig zur Verpressung der Ausgangsstoffe, den Wirkstoff alleine, oder den Wirkstoff zusammen mit einem der Zusatzstoffe, oder den Wirkstoff zusammen mit mehreren der Zusatzstoffe oder zusammen mit sämtlichen Zusatzstoffen, trockengranulieren.

Aus der derart erhaltenen Mischung der Ausgangsstoffe lassen sich Filmtabletten mit einem Wirkstoffgehalt von 25 Gew.-% bis 80 Gew.-% herstellen, welche sowohl eine genügende Härte, eine genügende Abriebfestigkeit und eine genügende Bioverfügbarkeit des Wirkstoffs, als auch eine genügende Lagerstabilität, insbesondere eine genügende Stabilität gegen Feuchtigkeit, aufweisen. Dies gilt insbesondere auch bei Verwendung von Imatinib-Mesylat in der alfaKristallform.

Die vorliegende Erfindung ist in den Patentansprüchen definiert. Insbesondere betrifft die vorliegende Erfindung eine Filmtablette, bestehend aus einem Tablettenkern mit einem Filmüberzug oder ein Granulat, welche als Wirkstoff eine Pyridylpyrimidinverbindung oder ein pharmazeutisch annehmbares Salz dieser Verbindung, vorzugsweise Imatinib oder ein pharmazeutisch annehmbares Salz von Imatinib, vorzugsweise Imatinib-Monomethansulfonat, enthalten, dadurch gekennzeichnet, dass (i) die Tablettenkerne und die Granulate mittels Verpressung der Ausgangsstoffe, d.h. mittels Verpressung des Wirkstoffs zusammen mit den Zusatzstoffen im Gemisch, hergestellt wurden, und vorgängig zurVerpressung derAusgangsstoffe, mindestens einer der Ausgangsstoffe trockengranuliert, vorzugsweise kompaktiert, worden ist; (ii) die Tablettenkerne und Granulatkerne den Wirkstoff in einem Anteil von 25 Gew.-% bis 80 Gew.-%, bezogen auf das Gesamtgewicht der Tablettenkerne bzw. der Granulatkerne und zusammen mit (iii) mindestens einem Füll- und Bindemittel enthalten, sowie gegebenenfalls weitere Zusatzstoffe aufweisen.

Vorzugsweise liegt die durchschnittliche Korngrössenverteilung von mindestens 80 % der Wirkstoffmenge im Bereich von 0.01 mm bis 1.0 mm, vorzugsweise im Bereich von 0.05 mm bis 1.0 mm.

Im Rahmen der vorliegenden Erfindung bedeutet der Ausdruck "Filmtablette" einen mit einem Filmüberzug versehenen Tablettenkern. DerAusdruck "Granulat" bedeutet hierin ein unbefilmtes Granulat oder mit einem Filmüberzug versehener Granulatkern, vorzugsweise ein unbefilmtes Granulat.

Verpressung der Ausgangsstoffe bedeutet, dass alle Komponenten der Ausgangsstoffe im Gemisch anschliessend an die erfindungsgemässe Trockengranulierung, gegebenenfalls gesiebt, direkt zu einer Tablette oder zum Granulat, verarbeitet werden.

Trockengranulierung der Ausgangsstoffe bedeutet, dass man mindestens einen der Ausgangsstoffe verdichtet, vorzugsweise kompaktiert bzw. trockengranuliert, vorzugsweise mit Walzen kompaktiert bzw. verdichtet, derart, dass sämtliche Ausgangsstoffe im Gemisch eine fliessfähige Mischung ergeben. Vorzugsweise werden erfindungsgemäss mindestens der Wirkstoff zusammen mit mindestens einem der Zusatzstoffe, oder den Wirkstoff zusammen mit mehreren ausgewählten Zusatzstoffen oder zusammen mit sämtlichen Zusatzstoffen, trockengranuliert.

Die bei der Trockengranulierung bzw. Kompaktierung erhaltenen Presslinge (auch "Schülpen" oder "Kompaktate" genannt) werden zu Granulatkörpern gebrochen, gegebenenfalls gesiebt, und weiter zu Tabletten oder Granulaten verarbeitet. Dabei werden überraschenderweiseTabletten und Granulate mit den erforderlichen Eigenschaften bezüglich Härte, Zerfall, Auflösegeschwindigkeit, und Lagerstabilität erhalten, insbesondere auch bei Verwendung von Imatinib-Mesylat in der alfa-Kristallform.

Die Trockengranulierung bzw. Kompaktierung an sich ist bekannt und lässt sich z.B. in einem Walzenkompaktor, z.B. der Marke Gerteis®, Alexanderwerk®, oder Powtec®, durchführen. Solche Apparaturen granulieren bzw. kompaktieren in der Regel bei Drucken im Bereich von 10-300 bar, vorzugsweise im Bereich von etwa 30-100 bar. Vorzugsweise genügt ein Druck von etwa 40 bis 80 bar (entsprechend etwa 2.8 KN/cm bis etwa 5.5 KN/cm). Der angewendete Druck für die optimale Kompaktierung bzw. Verdichtung ist in der Regel von der verwendeten Apparatur abhängig und kann vom Fachmann ohne weiteres optimal eingestellt werden, so dass die erfindungsgemässe fliessfähige Mischung der Komponenten erhalten wird.

Die vorliegende Erfindung betrifft auch Pulvermischungen, Tablettenkerne und Granulate, welche nicht mit einem Filmüberzug versehen sind, und welche als Zwischenprodukte für die Herstellung der erfindungsgemässen Filmtabletten und befilmten Granulate eingesetzt werden. Die vorliegende Erfindung betrifft auch Verfahren zur Herstellung der erfindungsgemässen Filmtabletten und befilmten Granulate.

Die vorliegende Erfindung betrifft im Weiteren die Verwendung der erfindungsgemässen Filmtabletten und Granulate als Tyrosin-Kinase-Hemmer, und insbesondere als Heilmittel zur Behandlung von Leukämie und andern an sich bekannten Indikationen.

Die erfindungsgemässe Filmtablette und das erfindungsgemässe Granulat können irgend eine der an sich bekannten Filmtabletten- oder Granulatformen aufweisen. Bevorzugt ist die Verwendung als Filmtablette.

Neben der freien Base der Pyridylpyrimidinverbindung, d.h. die Verbindung wurde nicht vorgängig in ein Salz umgewandelt, enthalten die erfindungsgemässen Tablettenkerne oder Granulate vorzugsweise als Beimischung, eine anorganische, pharmazeutisch annehmbare, sauer reagierende Verbindung, vorzugsweise eine Säure, vorzugsweise eine organische Carbonsäure oder organische Sulfonsäure, vorzugsweise Methansulfonsäure. Bevorzugt enthalten die genannten Kerne, die Pyridylpyrimidinverbindungen in Salzform, vorzugsweise als organisches, pharmazeutisch annehmbares Salz, wie beispielsweise das vorgehend genannte Mesylatsalz.

Die Tablettenkerne oder die Granulatkerne können unabhängig voneinander mit einem dünnen Filmüberzug versehen werden. Aus dem Tablettenkern wird derart eine Filmtablette erhalten, welche ausgezeichnete physikalische Eigenschaften aufweist. Werden die Granulatkerne befilmt, so können die erhaltenen befilmten Granulate unverändert, beispielsweise in Sachets oder Hartgelatinekapseln abgefüllt, direkt verwendet oderzu Tabletten direktverpresst werden. Eine derart erhaltene Filmtablette kann gegebenenfalls nochmals befilmt werden; eine weitere Befilmung der Filmtablette ist jedoch in der Regel nicht nötig. Für die Verpressung zu Tabletten werden vorzugsweise nicht-befilmten Granulatkerne verwendet. Diese können auch unverändert, d.h. nichtbefilmt, in Sachets oder Hartgelatinekapseln abgefüllt, direkt verwendet werden.

Die Tablettenkerne und Granulatkerne können mit an sich bekannten Methoden befilmtwerden, beispielsweise in einer Befilmungsapparatur (Coater) oder im Wirbelschichtverfahren. Solche bekannte Coating Apparaturen sind beispielsweise die kommerziell erhältlichen Coating Apparaturen von Glatt® oder Manesti®.

Überraschenderweise zeigte sich, dass die kristalline alfa-Form des Wirkstoffs für die Verwendung im erfindungsgemässen Verfahren besonders gut geeignet ist.

Die Filmtablettenkerne und Granulatkerne enthalten den Wirkstoff in einem Anteil von 25 Gew.-% bis 80 Gew.-%, vorzugsweise in einem Anteil von 30 Gew.-% bis 80 Gew.-%, vorzugsweise in einem Anteil von 40 Gew.-% bis 75 Gew.-%, vorzugsweise in einem Anteil von 50 Gew.-% bis 70 Gew.-%, bezogen auf das Gesamtgewicht des Tablettenkerns bzw. des Granulatkerns. Dabei beträgt die Menge an Wirkstoff pro Verabreichungseinheit, z.B. in einer Filmtablette, in einem Sachet oder einer Hartgelatinekapsel, jeweils etwa 50 mg bis 1000 mg, vorzugsweise 100 mg, 200 mg, 300 mg, 400 mg oder 600 mg, pro Verabreichungseinheit.

Die Tablettenkerne und Granulatkerne enthalten mindestens ein Füll- und Bindemittel. Eine solche Verbindung (oder ein Gemisch solcher Verbindungen) erfüllt in trockenem Zustand gleichzeitig die Funktion eines Füllmittels als auch die Funktion eines Bindemittels. Dabei haben diese Verbindungen einen Wassergehalt, mit welchem diese üblicherweise als "trocken" gekennzeichnet werden. In diesem Sinne liegt der Wassergehalt des Füll- und Bindemittels vorzugsweise im Bereich von 0.5 Gew.-% bis 10.0 Gew.-%, vorzugsweise im Bereich von 0.5 Gew.-% bis 5.0 Gew.-%, berechnet auf das Gesamtgewicht des Füll- und Bindemittels.

Geeignete Füll- und Bindemittel sind vorzugsweise ausgewählt aus der Gruppe enthaltend Zucker, Zuckeralkohole, polymere Glycoside, und anorganische Verbindungen (Salze). Zucker sind beispielsweise Saccharose und Lactose als Monohydrat oder in wasserfreier Form. Zuckeralkohole sind beispielsweise Mannitol (z.B. Pearlitol®, z.B. Pearlitol 400DC), Xylitol, Sorbitol. Polymere Glycoside sind beispielsweise Maltodextrin, (mikrokristalline) Cellulose und Stärken verschiedenen Ursprungs, wie z. B. Maisstärke. Geeignete Salze sind beispielsweise Kalziumhydrogenphosphat (als Dihydrat oder in wasserfreier Form), Kalziumsilikate und Natriumcarbonat. Bevorzugte Füll- und Bindemittel sind Kalziumsilikate, Cellulose und Stärke.

Geeignete Füll- und Bindemittel sind auch an sich bekannte modifizierte Stärken, modifizierte Cellulose, modifizierte Zucker, modifizierte Zuckeralkohole und modifizierte Lactose; Gelatine (auch als Hydrolsat), Gummi arabicum, gelatinisierte Stärke und Povidone (Hydroxpovidone). Modifizierte Cellulose umfasst vor allem Carboxymethylcellulose, Hydroxyethylcellulose, Hydroxypropylmethylcelulose (Hypromellose), Methylcellulose und Propylcellulose. Bevorzugt sind Propylcellulose (Hypromellose) und Povidone, insbesondere Hydroxypovidon.

Bevorzugt verwendet man die genannten Füll- und Bindemittel in Konzentrationen von 75 Gew.-% bis 20 Gew.-%, vorzugsweise von 70 Gew.-% bis 20 Gew.-%, vorzugsweise in einem Anteil von 60 Gew.-% bis 25 Gew.-%, und vorzugsweise in einem Anteil von 50 Gew.-% bis 30 Gew.-%, bezogen auf das Gesamtgewicht des Tablettenkerns bzw. des Granulatkerns in Ergänzung zum anwesenden Wirkstoff, wobei die Summe des Wirkstoffs und des anwesenden Füll- und Bindemittels sich auf 100 Gew.-% ergänzen.

Es ist jedoch von Vorteil, dass gegebenenfalls weitere Zusatzstoffe anwesend sind, welche den Gewichtsanteil des Füll- und Bindemittels entsprechend vermindern. In diesem Sinne können die Kerne weitere Zusatzstoffe enthalten. Solche Zusatzstoffe sind beispielsweise Sprengmittel, Fliessreguliermittel, Schmiermittel und Formtrennmittel. Bevorzugt ist die Kombination von Wirkstoff, Füll- und Bindemittel und Sprengmittel. Weiter bevorzugt ist auch die Kombination Wirkstoff, Füll- und Bindemittel, Sprengmittel und Fliessreguliermittel.

Geeignete Sprengmittel sind beispielsweise Crospovidon und Croscarmelose in der Form der Kalzium- oder Natriumsalze, Stärken und modifizierte Stärken, wie beispielsweise Maisstärke in pregelatinisierter Form oder als Natriumglycolat, Kalziumsilicat und niedrig substituierte Propylcellulose (L-HPC). Bevorzugte Sprengmittel sind Croscarmelose Natrium und Stärke-Natriumglycolat. Diese Sprengmittel setzt man in Mengen von 0.5 Gew.-% bis 10.0 Gew.-%, vorzugsweise in Mengen von 0.5 Gew.-% bis 5.0 Gew.-%, zu, bezogen auf das Gesamtgewicht des Tablettenkerns bzw. des Granulatkerns.

Geeignete Fliessreguliermittel sind beispielsweise hochdisperses Siliziumdioxid, hochdisperses Aluminiumdioxid, Kalziumsilikate und Talk. Bevorzugt sind jedoch Zusammensetzungen, welche kein Fliessreguliermittel enthalten.

Geeignete Schmier- und Form-Trennmittel sind Kalziumstearat, Magnesiumstearat, Stearinsäure, Natriumstearylfumarat, Talkum, Natriumbenzoat, Polyalkylenoxide, mikronisiertes Leucin, Glycerinmonostearat oder hydriertes Rizinusöl. Bevorzugt sind Kalziumstearat, Magnesiumstearat, und Natrium-stearyl-fumarat. Bevorzugte Mengen sind beispielsweise 0.1 bis 5 Gew.-%, vorzugsweise 0.5 bis 3 Gew.-%, bezogen auf das Gesamtgewicht des Tablettenkerns bzw. des Granulatkerns. Bevorzugte Polyalkylenoxide für die Verwendung als Schmier- und Form-Trennmittel sind z.B. Polyethylenoxide, Polypropylenoxide, Polyethylen-/propylenoxide. Hiervon bevorzugt ist Polyethylenglykol (z.B. Macrogol® 6000).

Die Tablettenkerne, und gegebenenfalls auch die Granulatkeme, sind mit einem Filmüberzug versehen. Dieser Filmüberzug enthält mindestens eine Verbindung, welche ausgewählt ist aus der Gruppe enthaltend filmbildende Substanzen, beispielsweise Hydroxypropyl-Methylcellulose (Hypromellose), Propylcellulose, Methylcellulose, Polyvinylalkohol, Polymethacrylate und Carrageen, wobei gegebenenfalls weitere Hilfsstoffe, wie Weichmacher, Gleitmittel und Farbstoffe, anwesend sein können. Bevorzugte Weichmacher sind Polyethylenglykol (Macrogol®, z.B. Macrogol 6000), Triethylcitrat, Triacetin.

Der Filmüberzug kann Stoffe zur besseren Haftung des Films, vorzugsweise Lactose und/oder Stearinsäure, sowie Trennmittel/Antiklebemittel, vorzugsweise Talkum und/oder Glycerinmonostearat sowie Farbstoffe (Pigmente) enthalten. Es können auch "instant"-Mischungen (premix) aus diesen Hilfsstoffen eingesetzt werden. Solche stabilisierende Zusatzstoffe entsprechen dem Stand der Technik und können vom Fachmann ohne weiteres auch in der vorliegenden Erfindung verwendet werden.

Die Dicke der Beschichtung wird durch die aufgetragene Lackmenge von etwa 1.0 bis 5.0 Gew.-% bezogen auf das Gewicht des Tablettenkerns bzw. gegebenenfalls des Granulatkerns bestimmt.

Die durchschnittliche Korngrössenverteilung des Wirkstoffs, von mindestens 80 % des Wirkstoffs, liegt im Bereich von 0.01 mm bis 1.0 mm, vorzugsweise im Bereich von 0.1 mm bis 0.8 mm, und insbesondere im Bereich von 0.1 mm bis 0.6 mm.

Die vorliegende Erfindung betrifft auch ein Verfahren zur Herstellung der erfindungsgemässen Filmtablette und des erfindungsgemässen Granulats, welches dadurch gekennzeichnet ist, dass man (i) die Tablettenkerne und die Granulate mittels Verpressung der Ausgangsstoffe, d.h. mittels Verpressung des Wirkstoffs zusammen mit den Zusatzstoffen im Gemisch, herstellt, und vorgängig zur Verpressung der Ausgangsstoffe mindestens einen der Ausgangsstoffe trockengranuliert, vorzugsweise verdichtet bzw. kompaktiert, und die erhaltenen Tablettenkerne und Granulate gegebenenfalls mit einem Filmüberzug überzieht; wobei (a) die Ausgangsstoffe den Wirkstoff und mindestens eine als Füll- und Bindemittel wirkende Verbindung, sowie gegebenenfalls weitere Zusatzstoffe im Gemisch enthalten; (b) der Wirkstoff in einem Anteil von 25 Gew.-% bis 80 Gew.-%, bezogen auf das Gesamtgewicht der Ausgangsstoffe vorliegt.

Vorzugsweise liegt die durchschnittliche Korngrössenverteilung von mindestens 80 % der Wirkstoffmenge im Bereich von 0.01 mm bis 1.0 mm, vorzugsweise im Bereich von 0.05 mm bis 1.0 mm.

Bevorzugt ist das Verfahren, worin man vorzugsweise mit Walzen trockengranuliert bzw. kompaktiert. Vorzugsweise werden erfindungsgemäss mindestens der Wirkstoff zusammen mit mindestens einem der Zusatzstoffe, oder den Wirkstoff zusammen mit mehreren ausgewählten Zusatzstoffen oder zusammen mit sämtlichen Zusatzstoffen, trockengranuliert. Vorzugsweisewird mit einem Walzenkompaktorbei Drucken im Bereich von 10-300 bar, vorzugsweise im Bereich von etwa 30-100 bar und vorzugsweise bei einem Druck im Bereich von etwa 40-80 bar trockengranuliert.

Die folgenden Beispiele erläutern die Erfindung.

### Beispiel 1 (Direkttablettierung)

Die Komponenten gemäss der folgenden Tabellen 1, 2, 3, 4, 5, 6 und 7 (bezeichnet als Granulat-Komponenten und Endgemisch oder Pulvergemisch) werden in an sich bekannter Weise in einem Freifallmischer der Marke Zancaetta® gemischt und das erhaltene Trockengemisch mit einer Presse der Marke Fette® bei einem Druck von 5.0 bis 20 KN (Kilo-Newton) bei Raumtemperatur zu Tablettenkernen und zu Granulatkernen verpresst. Anschliessend werden die erhaltenen Tablettenkerne in einer Befilmungsapparatur der Firma Glatt, gemäss Beispiel 3, befilmt.

### Beispiel 2 (Kompaktierung)

Der Wirkstoff allein, oder der Wirkstoff zusammen mit dem Füll- und Bindemittel, werden mittels eines Walzenkompaktors oder einer Tablettenpresse, bei einem Druck von etwa 5 KN/cm (entsprechend etwa 70 bar) kompaktiert. Die Kompaktate werden gebrochen, mit einem Frewitt®-Sieb gesiebt, und mit den weiteren Hilfsstoffen, d.h. den Komponenten der Endmischung, entsprechend den Tabellen 1,2,3, 4, 5, 6 und 7 gemischt. Das erhaltene Trockengemisch wird mit einer Tablettenpresse zu Tablettenkernen und Granulatkernen, analog zu Beispiel 1, verpresst.

### Beispiel 3 (Befilmung der Filmtablettenkerne)

Die Tablettenkerne und Granulatkerne, erhalten gemäss den Beispielen 1 und 2, werden in einem Glatt® Coater mit einer wässerigen Suspension oder Lösung derfilmbildenden Komponenten, gemäss den Tabellen 1-7, befilmt.

**Tabelle 1**

| Nr. | Komponenten | Zusammensetzung pro Einheit (mg) | Anteil (%) |
|---|---|---|---|
| | | | |

| | Granulat: | | |
|---|---|---|---|
| 1 | Imatinib mesylate * | 119.500 | 61.3 |
| 2 | Microcrystalline Cellulose | 25.000 | 12.8 |
| 4 | Crospovidone | 20.000 | 10.3 |
| 3 | Hypromellose (Hydroxypropylmethylcellulose) | 2.500 | 1.3 |
| | Endmischung: | | 0.0 |
| 2 | Microcrystalline cellulose | 11.100 | 5.7 |
| 4a | Crospovidone | 8.000 | 4.1 |

| Nr. | Komponenten | Zusammensetzung pro Einheit (mg) | Anteil (%) |
|---|---|---|---|
| 6 | Magnesium stearat | 1.400 | 0.7 |
| | Filmüberzug: | | |
| 7 | Opadry | 7.500 | 3.8 |
| | Gesamtgweicht | 195.000 | 100.0 |
| * bedeutet alfa- oder beta-Form, vorzugsweise die alfa-Form | | | |

**Tabelle 2**

| Nr. | Komponenten | Zusammensetzung pro Einheit (mg) | Menge (%) |
|---|---|---|---|
| | | | |

| | Granulat: | | |
|---|---|---|---|
| 1 | Imatinib mesylat* | 119.500 | 61.3 |
| 2 | Microcrystalline Cellulose | 25.000 | 12.8 |
| 3 | Povidone | 2.500 | 1.3 |
| 4 | Crospovidone | 20.000 | 10.3 |
| | Endmischung: | | 0.0 |
| 2 | Microcrystalline Cellulose | 9.850 | 5.1 |
| 4a | Crospovidone | 8.000 | 4.1 |
| 5 | Hochdisperses Siliciumdioxid | 1.250 | 0.6 |
| 6 | Magnesiumstearat | 1.400 | 0.7 |
| | Filmüberzug: | | |
| 7 | Opadry | 7.500 | 3.8 |
| | Gesamtgewicht | 195.000 | 100.0 |
| * bedeutet alfa- oder beta-Form, vorzugsweise die alfa-Form | | | |

**Tabelle 3**

| Nr. | Komponenten | Zusammensetzung pro Einheit (mg) | Anteil (%) |
|---|---|---|---|
| | | | |

| | Granulat: | | |
|---|---|---|---|
| 1 | Imatinib mesylat * | 119.500 | 61.3 |
| 2 | Calciumsilicat | 25.000 | 12.8 |
| 3 | Hypromellose (Hydroxypropylmethylcellulose) | 2.500 | 1.3 |
| | Endmischung: | | 0.0 |
| 4 | Talkum | 9.850 | 5.1 |
| 5 | Crospovidone | 28.000 | 14.4 |
| 6 | Hochdisperses Siliziumdioxid | 1.250 | 0.6 |
| 7 | Magnesium stearate | 1.400 | 0.7 |
| | Filmüberzug: | | |
| Nr. | Komponenten | Zusammensetzung pro Einheit (mg) | Anteil (%) |
| 8 | Opadry | 7.500 | 3.8 |
| | Gesamtgewicht | 195.000 | 100.0 |
| * bedeutet alfa- oder beta-Form, vorzugsweise die alfa-Form | | | |

**Tabelle 4**

| Nr. | Komponenten | Zusammensetzung pro Einheit (mg) | Anteil (%) |
|---|---|---|---|
| | | | |
| | Granulat: | | |
| 1 | Imatinib mesylat * | 119.500 | 61.3 |
| 2 | Maisstärke | 25.000 | 12.8 |
| 3 | Povidone | 2.500 | 1.3 |
| | Endmischung: | | 0.0 |
| 2a | Maisstärke | 9.850 | 5.1 |
| 4 | Crospovidone | 28.000 | 14.4 |
| 5 | Hochdisperses Siliciumdioxid | 1.250 | 0.6 |
| 6 | Magnesiumstearat | 1.400 | 0.7 |
| | Filmüberzug: | | |
| 7 | Opadry | 7.500 | 3.8 |
| | Gesamtgewicht | 195.000 | 100.0 |
| * bedeutet alfa- oder beta-Form, vorzugsweise die alfa-Form | | | |

**Tabelle 5**

| Nr. | Komponenten | Zusammensetzung pro Einheit (mg) | Anteil.(%) |
|---|---|---|---|
| | | | |
| | Granulat: | | |
| 1 | Imatinib mesylat * | 119.500 | 61.3 |
| 2 | Microcrystalline Cellulose | 25.000 | 12.8 |
| 3 | Povidone | 2.500 | 1.3 |
| | Endmischung: | | |
| 2a | Microcrystalline cellulose | 9.850 | 5.1 |
| 4 | Crospovidone | 28.000 | 14.4 |
| 5 | Silica, colloidal anhydrous | 1.250 | 0.6 |
| 6 | Calciumstearat | 1.400 | 0.7 |
| | Filmüberzug: | | |
| 7 | Coating (gemäss Beispiel 3) | 7.500 | 3.8 |
| | Gesamtgewicht | 195.000 | 100.0 |

| Nr. | Komponenten | Zusammensetzung pro Einheit (mg) | Anteil (%) |
|---|---|---|---|
| | | | |
| * bedeutet alfa- oder beta-Form, vorzugsweise die alfa-Form | | | |

**Tabelle 6**

| Nr. | Komponenten | Zusammensetzung pro Einheit (mg) | Anteil (%) |
|---|---|---|---|
| | | | |
| | Granulate: | | |
| 1 | Imatinib mesylate * | 119.500 | 61.3 |
| 2 | Microcrystalline cellulose | 25.000 | 12.8 |
| 3 | Hypromellose (Hydroxypropylmethylcellulose) | 2.500 | 1.3 |
| | Endmischung (Final blend) | | |
| 2 | Microcrystalline cellulose | 9.850 | 5.1 |
| 4 | Crospovidone | 28.000 | 14.4 |
| 5 | Silica, colloidal anhydrous | 1.250 | 0.6 |
| 6 | Sodium stearylfumarat | 1.400 | 0.7 |
| | Filmüberzug: | | |
| 7 | Opadry | 7.500 | 3.8 |
| | Gesamtgewicht | 195.000 | 100.0 |
| * bedeutet alfa- oder beta-Form, vorzugsweise die alfa-Form | | | |

**Tabelle 7**

| Nr. | Komponenten | Zusammensetzung pro Einheit (mg) | Anteil (%) |
|---|---|---|---|
| | Granulat: | | |
| 1 | Imatinib mesylate * | 119.500 | 61.3 |
| 2 | Microcrystalline Cellulose | 35.000 | 17.9 |
| 3 | Crospovidone | 30.000 | 15.4 |
| | Endmischung: | | |
| 4 | Hochdisperses Siliciumdioxid | 1.0 | 0.5 |
| 5 | Magnesium stearat | 2.0 | 1.0 |
| | Filmüberzug: | | |
| 6 | Opadry | 7.500 | 3.8 |
| | Gesamtgewicht | 195.000 | 100.0 |
| * bedeutet alfa- oder beta-Form, vorzugsweise die alfa-Form | | | |

### Beispiel 4 (Direkttablettierung)

717 g Imatinib Mesylat (jeweils alfa- oder beta-Form), 201.6 g MCC (Avicel® PH 200), 168 g Crospovidone (Plasdone® XL), 22.5 g Talkum und 7.5g colloidales Siliciumdioxid (Aerosil@ 200) werden in einem Edelstahtfass mit einem Turbula-Mischer während 10 Minuten gemischt, über ein 1.4 mm Sieb gesiebt und nochmals für 10 Minuten gemischt. Zu der erhaltenen Trockenmischung wird 8,4 g Magnesiumstearat gegeben und weitere 3 Minuten gemischt. Die Pulvermischung wird anschliessend auf einer Rundläufertablettenpresse (Korsch® XL100) mit einem Druck von 5 bis 25 KN zu Tablettenkernen mit 187.5 mg Gewicht tablettiert und dann in einem Coater (Glatt) mit einer wässrigen Opadry®-Lacksuspension befilmt.

### Beispiel 5 (Kompaktierung)

717 g gemahlenes Imatinib Mesylat (alfa-Modifikation), 74.1 g MCC (Avicel® PH 101), 100 g Crospovidone (Plasdone® XL) werden in einem Edelstahlfass mit einem Turbula-Mischer während 10 Minuten gemischt. Die Pulvermischung wird mittels eines Walzenkompaktors (Powtec® RC100x30) bei einem Druckvon ca. 3.5 KN/cm (entsprechend etwa 50 bar) zu Schülpen kompaktiert, die mittels eines Frewittsiebs über ein 1.4 mm Sieb gebrochen werden. Das erhaltene Granulat wird zusammen mit 150 g MCC (Avicel® PH 200), 68 g Crospovidone (Plasdone® XL)und 7.5g colloidales Siliciumdioxid (Aerosil@ 200) in ein Edelstahlfass gegeben und mit einem Turbula-Mischer während 10 Minuten gemischt, anschliessend wird 8,4 g Magnesiumstearat zuggegeben und für weitere 3 Minuten gemischt. Die Pulvermischung wird anschliessend auf einer Rundläufertablettenpresse bei einem Druck von 0.5 bis 15 KN/cm zu Tabletten mit 187.5 mg Gewicht (Korsch® XL100) tablettiert. Die erhaltenen Kerne werden anschliessend in einem Coater (Glatt®) mit einer wässrigen Lacksuspension (Opadry®) befilmt.

### Beispiel 6 (Kompaktierung)

239 g gemahlenes Imatinib Mesylat (alfa-Modifikation), 70 g MCC (Avicel® PH 101), 60 g Crospovidone (Plasdone®XL)werden in einem Edelstahlfass mit einemTurbula-Mischerwährend 10 Minuten gemischt. Die Pulvermischung mit einem Schüttvolumen von 226 ml wird mittels eines Walzenkompaktors (Powtec® RC 100x30) mit den aus Tabelle 8 ersichtlichen Einstellungen zu Schülpen kompaktiert, die mittels eines Frewittsiebs über ein 0.8 mm Sieb gebrochen werden. Der Feinanteil (< 0.3 mm) wird erneut bei einem Druck von ca.3.5 KN/cm (entsprechend 50 bar) kompaktiert. Das erhaltene Granulat mit einem Schüttvolumen von 168 bis 174 ml wird zusammen mit 2.0 g colloidalem Siliciumdioxid (Aerosil® 200) in ein Edelstahlfass gegeben und mit einem Turbula-Mischer während 10 Minuten gemischt, anschliessend wird 4.0 g Magnesium-stearat zuggegeben und für weitere drei Minuten gemischt. Die Pulvermischung wird anschliessend auf einer Rundläufer-Tablettenpresse (Fette® P1) bei einem Druck von 0.5 bis 15 KN zu Tabletten mit 187.5 mg Gewicht tablettiert. Die erhaltenen Kerne werden anschliessend in einem Coater (Glatt®) mit einer wässrigen Lacksuspension (Opadry®) befilmt. Die erhaltenen gewölbten Filmtabletten weisen folgende Eigenschaften auf: Durchmesser 9 mm, Höhe 3.5 mm, Härte durchschnittlich 98 N (in einem Bereich von 80 N bis 108 N),bei einer Zerfallszeit von 6:00 bis 7:40 Minuten und einer Freisetzung von ≥90 % nach 30 Minuten (900ml, 0.1 N HCl, paddle, 50 rpm, Apparatus 2 nach USP).

**Tabelle 8: Einstellungen des Walzenkompaktors**

| Nr. | Parameter | Eingestellter Wert |
|---|---|---|
| | | |
| 1 | Walzendrehzahl | 4 rpm |
| 2 | Förderschneckendrehzahl | 24 rpm |
| 3 | Walzendruck | 3.5 KN/cm |
| 4 | Siebmaschenweite | 0.8 mm |

### Beispiel 7 (Kompaktierung, Imatinib 400mg Filmtablette)

Nach den in Beispiel 6 angegebenen Bedingungen werden 956 g gemahlenes Imatinib Mesylat (alfa-Mdifikation), 280 g MCC (Avicel® PH 101), 240 g Crospovidone (Plasdone® XL gemischt und kompaktiert. Das erhaltene Trockengranulat wird zusammen mit 8.0 g colloidalem Siliciumdioxid (Aerosil@ 200) gemischt, anschliessend wird 16.0 g Magnesium-stearat zugegeben, gemischt und die erhaltene Mischung tablettiert. Die erhaltenen Tabletten weisen die folgenden Eigenschaften auf: Gewicht 750mg (entspr. 400mg Imatinib Base), oblong gewölbte Tabletten, Länge 18 mm, Breite 7.5 mm, Höhe 7.2 mm; Härte durchschnittlich 120 N. DieTabletten werden anschliessend noch mit einem Opadry®-Lack befilmt.

## Patentansprüche

1. Verfahren zur Herstellung einer Filmtablette, bestehend aus einem Tablettenkern mit einem Filmüberzug oder eines Granulats, welche als Wirkstoff Imatinib-Monomethansulfonat in der kristallinen alfa-Form enthalten, indem man die Tablettenkerne und die Granulate mittels Verpressung der Ausgangsstoffe herstellt, und diese Ausgangsstoffe den Wirkstoff und mindestens eine als Füllund Bindemittel wirkende Verbindung, sowie gegebenenfalls weitere Zusatzstoffe im Gemisch enthalten; **dadurch gekennzeichnet, dass** man vorgängig zur Verpressung der Ausgangsstoffe, den Wirkstoff zusammen mit mindestens einem der Zusatzstoffe trockengranuliert, und die erhaltenen Tablettenkerne, und gegebenenfalls die Granulate, mit einem Filmüberzug überzieht; wobei die derart hergestellten Tablettenkerne und Granulatkerne den Wirkstoff in einem Anteil von 25 Gew.-% bis 80 Gew.-%, bezogen auf das Gesamtgewicht der Tablettenkerne bzw. der Granulatkerne enthalten.

2. Verfahren nach Anspruch 1, **dadurch gekennzeichnet, dass** die durchschnittliche Korngrössenverteilung von mindestens 80 % der Wirkstoffmenge im Bereich von 0.01 mm bis 1.0 mm, vorzugsweise im Bereich von 0.05 mm bis 1.0 mm, liegt.

3. Verfahren nach Anspruch 1 oder 2, **dadurch gekennzeichnet, dass** man mit Walzen trockengranuliert.

4. Verfahren nach einem der Ansprüche 1-3, **dadurch gekennzeichnet, dass** man, vorgängig zur Verdichtung der Ausgangsstoffe, den Wirkstoff zusammen mit mehreren ausgewählten Zusatzstoffen oder zusammen mit sämtlichen Zusatzstoffen trockengranuliert.

5. Verfahren nach einem der Ansprüche 1-4, **dadurch gekennzeichnet, dass** man die Trockengranulierung in einem Walzenkompaktor, vorzugsweise bei Drucken im Bereich von 10-300 bar, vorzugsweise im Bereich von etwa 30-100 bar und vorzugsweise bei einem Druck von etwa 40 bis 80 bar (entsprechend 3.5 KN/cm bis etwa 5.5 KN/cm), durchführt.

6. Filmtablette oder Granulat hergestellt nach einem der Ansprüche 1-5, **dadurch gekennzeichnet, dass** der Filmtablettenkern bzw. Granulatkern den Wirkstoff in einem Anteil von 30 Gew.-% bis 80 Gew.-%, vorzugsweise in einem Anteil von 40 Gew.-% bis 75 Gew.-%, und vorzugsweise in einem Anteil von 50 Gew.-% bis 70 Gew.-%, bezogen auf das Gesamtgewicht des Tablettenkerns bzw. des Granulatkerns, enthalten.

7. Filmtablette oder Granulat hergestellt nach einem der Ansprüche 1-5, **dadurch gekennzeichnet, dass** die Filmtablette eine Verabreichungseinheit bildet und das Granulat als Verabreichungseinheit in einem Sachet oder einer Hartgelatinekapsel abgefüllt ist, und die Menge an Wirkstoff pro Verabreichungseinheit jeweils etwa 50 mg bis 1000 mg, vorzugsweise 100 mg, 200 mg, 300 mg, 400 mg oder 600 mg, beträgt.

8. Filmtablette oder Granulat hergestellt nach einem der Ansprüche 1-5, **dadurch gekennzeichnet, dass** der Wassergehalt des Füll- und Bindemittels im Bereich von 0.5 Gew.-% bis 10.0 Gew.-%, vorzugsweise im Bereich von 0.5 Gew.-% bis 5.0 Gew.-%, liegt, berechnet auf das Gesamtgewicht des Füll- und Bindemittels.

9. Filmtablette oder Granulat hergestellt nach einem der Ansprüche 1-5, **dadurch gekennzeichnet, dass** das Füll- und Bindemittel ausgewählt ist aus der Gruppe enthaltend Zucker, vorzugsweise Saccharose und/oder Lactose als Monohydrat oder in wasserfreier Form; Zuckeralkohole, vorzugsweise Mannitol, Xylitol und/oder Sorbitol; polymere Glycoside, vorzugsweise Maltodextrin, mikrokristalline Cellulose und/oder Stärken verschiedenen Ursprungs, vorzugsweise Maisstärke; und/oder anorganische Salze, vorzugsweise Kalziumhydrogenphosphat als Dihydrat oder in wasserfreier Form, Kalziumsilikate und/oder Natriumcarbonat, vorzugsweise Kalziumsilikat, Cellulose und/oder Stärke.

10. Filmtablette oder Granulat hergestellt nach einem der Ansprüche 1-5, **dadurch gekennzeichnet, dass** das Füll- und Bindemittel ausgewählt ist aus der Gruppe enthaltend an sich bekannte modifizierte Stärken; modifizierte Cellulose, vorzugsweise Carboxymethylcellulose, Hydroxyethylcellulose, Hydroxypropylmethylcelulose, Methylcellulose und/oder Propylcellulose, vorzugsweise Propylcellulose (Hypromellose); modifizierte Zucker, modifizierte Zuckeralkohole und modifizierte Lactose; Gelatine; Gummi arabicum; gelatinisierte Stärke; und/oder Povidone, vorzugsweise Hydroxypovidon.

11. Filmtablette oder Granulat hergestellt nach einem der Ansprüche 1-5, **dadurch gekennzeichnet, dass** das Füll- und Bindemittel in Konzentrationen von 75 Gew.-% bis 20 Gew.-%, vorzugsweise von 70 Gew.-% bis 20 Gew.-%, vorzugsweise von 60 Gew.-% bis 25 Gew.-%, und vorzugsweise von 50 Gew.-% bis 30 Gew.-%, bezogen auf das Gesamtgewicht des Tablettenkerns bzw. des Granulatkerns in Ergänzung zum anwesenden Wirkstoff anwesend ist, wobei die Summe des Wirkstoffs und des anwesenden Füll- und Bindemittels sich auf 100 Gew.-% ergänzen.

12. Filmtablette oder Granulat hergestellt nach einem der Ansprüche 1-5, **dadurch gekennzeichnet, dass** deren Tablettenkerne bzw. Granulatkerne weitere Zusatzstoffe enthalten, vorzugsweise ausgewählt aus der Gruppe enthaltend Sprengmittel, Fliessreguliermittel, Schmiermittel und Formtrennmittel, vorzugsweise eine Kombination von Wirkstoff, Füll- und Bindemittel und Sprengmittel, vorzugsweise eine Kombination von Wirkstoff, Füll- und Bindemittel, Sprengmittel und Fliessreguliermittel.

13. Filmtablette oder Granulat hergestellt nach einem der Ansprüche 1-5, **dadurch gekennzeichnet, dass** der Filmüberzug mindestens eine Verbindung enthält, welche ausgewählt ist aus der Gruppe enthaltend filmbildende Substanzen, vorzugsweise Hydroxypropyl-Methylcellulose, Propylcellulose, Methylcellulose, Polyvinylalkohol, Polymethacrylate und Carrageen, wobei gegebenenfalls weitere Hilfsstoffe, wie Weichmacher, Gleitmittel und Farbstoffe, anwesend sind.

14. Filmtablette oder Granulat hergestellt nach einem der Ansprüche 1-5, **dadurch gekennzeichnet, dass** die durchschnittliche Korngrössenverteilung von mindestens 80 % des Wirkstoffs im Bereich von 0.05 mm bis 1.0 mm, vorzugsweise im Bereich von 0.1 mm bis 0.8 mm, und insbesondere im Bereich von 0.1 mm bis 0.6 mm, liegt.

15. Tablettenkerne nach einem der Ansprüch 6-14, welche nicht mit einem Filmüberzug versehen sind als Zwischenprodukte für die Herstellung von Filmtabletten gemäss einem der Ansprüche 1-5.

16. Granulatkerne nach Anspruch 15, welche in Sachets oder Hartgelatinekapseln abgefüllt sind.

17. Filmtablette, welche durch Verpressung aus einem Granulat, erhalten gemäss einem der Ansprüche 1-5, hergestellt wurde.

18. Verwendung von Imatinib-Monomethansulfonat in der kristallinen alfa-Form zur Herstellung von Filmtabletten und Granulaten gemäss einem der Ansprüche 1-5, als Tyrosin-Kinase-Hemmer und als Heilmittel zur Behandlung von Leukämie und andern an sich bekannten Indikationen.

## Claims

1. A process for the preparation of a film-coated tablet, consisting of a tablet core having a film coating or of a granulate, which contain as active ingredient imatinib monomethanesulfonate in the crystalline alpha form, by producing the tablet cores and the granulate by pressing the starting materials, and the starting materials comprise the active ingredient and at least one compound acting as filling and binding agent, as well as optional further additives in admixture, **characterized in that** prior to pressing the starting materials, the active ingredient is dry-granulated together with at least one of the additives, and the resulting tablet cores, and optionally the granules, are coated with a film coating, wherein the tablet cores and granule cores so prepared contain the active agent in an amount of from 25 wt.% to 80 wt.%, based on the total weight of the tablet cores or granule cores.

2. Process according to claim 1, **characterised in that** the average particle size distribution of at least 80% of the amount of active ingredient is in the range from 0.01 mm to 1.0 mm, preferably in the range from 0.05 mm to 1.0 mm.

3. A process according to claim 1 or 2, **characterized in that** the dry granulation is carried out with rollers.

4. A process according to any one of claims 1 to 3, **characterized in that** prior to compressing the starting materials, the active ingredient is dry-granulated together with multiple additives or together with all additives.

5. A process according to any one of claims 1 to 4, **characterized in that** the dry granulation is carried out in a roller compactor, preferably with pressing in the range of 10 - 300 bar, preferably in the range of about 30 - 100 bar and preferably at a pressure of about 40 to 80 bar (corresponding to 3.5 KN/cm to about 5.5 KN/cm).

6. Film-coated tablet or granules prepared according to any one of claims 1 to 5, **characterised in that** the film-coated tablet core or granule core contains the active ingredient in an amount of from 30 wt.% to 80 wt.%, preferably in an amount of from 40 wt.% to 75 wt.%, and preferably in an amount of from 50 wt.% to 70 wt.%, based on the total weight of the tablet core or granule core.

7. Film-coated tablet or granules prepared according to any one of claims 1 to 5, **characterised in that** the film-coated tablet forms a dosage unit and the granules are introduced into a sachet or hard gelatin capsule as a dosage unit, and the amount of active ingredient per dosage unit is in each case approximately from 50 mg to 1000 mg, preferably 100 mg, 200 mg, 300 mg, 400 mg or 600 mg.

8. Film-coated tablet or granules prepared according to any one of claims 1 to 5, **characterised in that** the water content of the filling and binding agent is in the range from 0.5 wt.% to 10.0 wt.%, preferably in the range from 0.5 wt.% to 5.0 wt.%, based on the total weight of the filling and binding agent.

9. Film-coated tablet or granules prepared according to any one of claims 1 to 5, **characterised in that** the filling and binding agent is selected from the group containing sugars, preferably sucrose and/or lactose as the monohydrate or in anhydrous form; sugar alcohols, preferably mannitol, xylitol and/or sorbitol; polymeric glycosides, preferably maltodextrin, microcrystalline cellulose and/or starches of various origins, preferably maize starch; and/or inorganic salts, preferably calcium hydrogen phosphate as the dihydrate or in anhydrous form, calcium silicates and/or sodium carbonate, preferably calcium silicate, cellulose and/or starch.

10. Film-coated tablet or granules prepared according to any one of claims 1 to 5, **characterised in that** the filling and binding agent is selected from the group containing the following compounds known *per se*: modified starches; modified celluloses, preferably carboxymethylcellulose, hydroxyethylcellulose, hydroxypropylmethylcellulose, methylcellulose and/or propylcellulose, preferably propylcellulose (hypromellose); modified sugars, modified sugar alcohols and modified lactose; gelatin; gum arabic; gelatinised starch; and/or povidones, preferably hydroxypovidone.

11. Film-coated tablet or granules prepared according to any one of claims 1 to 5, **characterised in that** the filling and binding agent is present in concentrations of from 75 wt.% to 20 wt.%, preferably from 70 wt.% to 20 wt.%, preferably from 60 wt.% to 25 wt.%, and preferably from 50 wt.% to 30 wt.%, based on the total weight of the tablet core or granule core, in addition to the active ingredient that is present, the sum of the active ingredient and of the filling and binding agent that is present being 100 wt.%.

12. Film-coated tablet or granules prepared according to any one of claims 1 to 5, **characterised in that** the tablet cores or granule cores thereof comprise further additives, preferably selected from the group containing disintegrators, flow regulators, lubricants and mould release agents, preferably a combination of active ingredient, filling and binding agent and disintegrator, preferably a combination of active ingredient, filling and binding agent, disintegrator and flow regulator.

13. Film-coated tablet or granules prepared according to any one of claims 1 to 5, **characterised in that** the film coating comprises at least one compound selected from the group containing film-forming substances, preferably hydroxypropylmethylcellulose, propylcellulose, methylcellulose, polyvinyl alcohol, polymethacrylates and carrageen, further auxiliary substances, such as plasticisers, lubricants and colourings, optionally being present.

14. Film-coated tablet or granules prepared according to any one of claims 1 to 5, **characterised in that** the average particle size distribution of at least 80% of the active ingredient is in the range from 0.05 mm to 1.0 mm, preferably in the range from 0.1 mm to 0.8 mm and in particular in the range from 0.1 mm to 0.6 mm.

15. Tablet cores according to any one of claims 6 to 14 which are not provided with a film coating, as intermediates for the preparation of film-coated tablets according to any one of claims 1 to 5.

16. Granule cores according to claim 15 which have been introduced into sachets or hard gelatin capsules.

17. Film-coated tablet which has been prepared by pressing from granules obtained according to any one of claims 1 to 5.

18. Use of imatinib monomethanesulfonate in the crystalline alpha form in the preparation of film-coated tablets and granules according to any one of claims 1 to 5, as a tyrosine kinase inhibitor and as a medicament for the treatment of leukaemia and other indications known *per se.*

## Revendications

1. Procédé de fabrication d'un comprimé pelliculé, composé d'un noyau de comprimé pourvu d'un film d'enrobage, ou d'un granulé, lesquels contiennent comme principe actif du monométhane-sulfonate d'imatinib sous forme alpha cristalline, procédé dans lequel les noyaux de comprimé et les granulés sont fabriqués par pressage des substances de départ et ces substances de départ contiennent le principe actif et au moins un composé, utilisé comme agent de remplissage et liant, ainsi qu'éventuellement d'autres additifs dans le mélange ; **caractérisé en ce que**, avant le pressage des substances de départ, le principe actif est granulé à sec conjointement avec au moins un des additifs, et les noyaux de comprimé obtenus, et éventuellement les granulés, sont enrobés avec un film d'enrobage ; les noyaux de comprimé et les noyaux de granulé ainsi fabriqués contiennent le principe actif dans une proportion de 25% en poids à 80% en poids, sur la base du poids total des noyaux de comprimé ou des noyaux de granulé.

2. Procédé selon la revendication 1, **caractérisé en ce que** la répartition granulométrique moyenne d'au moins 80 % de la quantité de substance active est comprise entre 0.01 mm et 1.0 mm, de préférence comprise entre 0.05 mm et 1.0 mm.

3. Procédé selon la revendication 1 ou 2, **caractérisé en ce que** la granulation à sec est effectuée au moyen de rouleaux.

4. Procédé selon l'une des revendications 1 à 3, **caractérisé en ce que**, avant le pressage des substances de départ, le principe actif est granulé à sec conjointement avec plusieurs additifs sélectionnés ou conjointement avec la totalité des additifs.

5. Procédé selon l'une des revendications 1 à 4, **caractérisé en ce que** la granulation à sec est effectuée dans un compacteur à cylindres, de préférence à des pressions de l'ordre de 10 à 300 bar, de préférence de l'ordre d'environ 30 à 100 bar et de préférence à une pression d'environ 40 à 80 bar (ce qui correspond à une pression de 3,5 KN/cm à environ 5,5 KN/cm).

6. Comprimé pelliculé ou granulé fabriqué selon l'une quelconque des revendications 1 à 5, **caractérisé en ce que** le noyau du comprimé pelliculé resp. le noyau du granulé comprend la substance active dans une proportion de 30 % en poids à 80 % en poids, de préférence dans une proportion de 40 % en poids à 75 % en poids, et de préférence dans une proportion de 50 % en poids à 70 % en poids, calculée par rapport au poids total du noyau de comprimé resp. du noyau de granulé.

7. Comprimé pelliculé ou granulé fabriqué selon l'une quelconque des revendications 1 à 5, **caractérisé en ce que** le comprimé pelliculé forme une unité d'administration et le granulé est rempli en tant qu'unité d'administration dans un sachet ou une capsule de gélatine dure, et la quantité de substance active par unité d'administration s'élève à chaque fois à environ 50 mg à 1000 mg, de préférence 100 mg, 200 mg, 300 mg, 400 mg ou 600 mg.

8. Comprimé pelliculé ou granulé fabriqué selon l'une quelconque des revendications 1 à 5, **caractérisé en ce que** la teneur en eau de l'agent de remplissage et liant est de l'ordre de 0.5 % en poids à 10.0 % en poids, de préférence de l'ordre de 0.5 % en poids à 5.0 % en poids, calculée par rapport au poids total de l'agent de remplissage et liant.

9. Comprimé pelliculé ou granulé fabriqué selon l'une quelconque des revendications 1 à 5, **caractérisé en ce que** l'agent de remplissage et liant est choisi dans le groupe comprenant des sucres, de préférence saccharose et/ou lactose sous forme de monohydrate ou sous forme anhydre ; des alcools de sucre, de préférence mannitol, xylitol et/ou sorbitol ; des glycosides polymériques, de préférence maltodextrine, cellulose microcristalline et/ou des amidons de différentes origines, de préférence de l'amidon de maïs ; et/ou des sels inorganiques, de préférence de l'hydrogénophosphate de calcium sous forme de dihydrate ou sous forme anhydre, des silicates de calcium et/ou du carbonate de sodium, de préférence silicate de calcium, cellulose et/ou amidon.

10. Comprimé pelliculé ou granulé fabriqué selon l'une quelconque des revendications 1 à 5, **caractérisé en ce que** l'agent de remplissage et liant est choisi dans le groupe comprenant des amidons modifiés connus en soi ; des celluloses modifiées, de préférence cellulose de carboxyméthyle, cellulose d'hydroxyéthyle, cellulose d'hydroxypropylméthyle, cellulose de méthyle et/ou cellulose de propyle, de préférence cellulose de propyle (hypromellose) ; des sucres modifiés, des alcools de sucre modifiés et du lactose modifié ; gélatine ; gomme arabique ; amidon gélatinisé ; et/ou povidone, de préférence hydroxypovidone.

11. Comprimé pelliculé ou granulé fabriqué selon l'une quelconque des revendications 1 à 5, **caractérisé en ce que** l'agent de remplissage et liant est présent dans des concentrations de 75 % en poids à 20 % en poids, de préférence de 70 % en poids à 20 % en poids, de préférence de 60 % en poids à 25 % en poids, et de préférence de 50 % en poids à 30 % en poids, par rapport au poids total du noyau du comprimé resp. du noyau du granulé en complément à la substance active présente, où la somme de la substance active et de l'agent de remplissage et liant présent se montent à 100 % en poids.

12. Comprimé pelliculé ou granulé fabriqué selon l'une quelconque des revendications 1 à 5, **caractérisé en ce que** les noyaux des comprimés resp. les noyaux des granulés comprennent d'autres additifs, de préférence choisis dans le groupe comprenant des agents de désintégration, des agents d'écoulement, des lubrifiants et des agents de démoulage, de préférence une combinaison de substance active, agent de remplissage et liant et agent de désintégration, de préférence une combinaison de substance active, agent de remplissage et liant, agent de désintégration et agent d'écoulement.

13. Comprimé pelliculé ou granulé fabriqué selon l'une quelconque des revendications 1 à 5, **caractérisé en ce que** l'enrobage comprend au moins un composé qui est choisi dans le groupe comprenant des substances filmogènes, de préférence cellulose d'hydroxypropylméthyle, cellulose de propyle, cellulose de méthyle, polyvinylalcool, polyméthacrylates et carraghénane, où d'autres adjuvants, tels que des plastifiants, des lubrifiants et des colorants, sont optionnellement présents.

14. Comprimé pelliculé ou granulé fabriqué selon l'une quelconque des revendications 1 à 5, **caractérisé en ce que** la répartition granulométrique moyenne d'au moins 80 % de la substance active est comprise entre 0.05 mm et 1.0 mm, de préférence comprise entre 0.1 mm et 0.8 mm, et en particulier comprise entre 0.1 mm et 0.6 mm.

15. Noyaux de comprimés selon l'une quelconque des revendications 6 à 14 qui ne sont pas munis d'un enrobage en tant que produits intermédiaires pour la fabrication de comprimés pelliculés selon l'une quelconque des revendications 1 à 5.

16. Noyaux de granulés selon la revendication 15, qui sont remplis dans des sachets ou des capsules de gélatine dure.

17. Comprimé pelliculé, fabriqué par pressage à partir d'un granulé obtenu selon l'une quelconque des revendications 1 à 5.

18. Utilisation du monométhane sulfonate d'imatinib sous forme cristalline alpha pour la fabrication de comprimés pelliculés et de granulés selon l'une quelconque des revendications 1 à 5, en tant qu'inhibiteur de tyrosine kinase et en tant que médicament pour le traitement de la leucémie et d'autres indications connues en soi.
